# EUROPÄISCHE PATENTANMELDUNG

(11) **EP 2 689 803 A2**
(43) Veröffentlichungstag der Anmeldung: **29.01.2014**
(21) Anmeldenummer: 13177192.5
(22) Anmeldetag: 19.07.2013
(51) Int. Cl.: A61Q 5/10, A61K 8/898

(54) **Pflegende Haarfärbemittel mit Blockcopolymeren**

(30) Priorität: 27.07.2012 DE 102012213250
(71) Anmelder: Henkel AG&Co. KGAA, 40589 Düsseldorf (DE)
(72) Erfinder: Schweinsberg, Matthias, 22769 Hamburg (DE); Grosjacques, Camille, DE-20255 Hamburg (DE); Bietz, Susanne, 25336 Elmshorn (DE); Hoepfner, Stefan, 22523 Hamburg (DE); Rohweder, Sandra, 25436 Heidgraben (DE); Manneck, Hartmut, 23858 Barnitz (DE)

(57) **Zusammenfassung**

Gegenstand der Erfindung ist daher eine Zusammensetzung zur Farbveränderung keratinischer Fasern, enthaltend mindestens eine Verbindung aus der Gruppe der Oxidationsfarbstoffvorprodukte, der direktziehenden Farbstoffe, Wasserstoffperoxid oder deren Mischungen und mindestens ein Silicon-Blockcopolymer, enthaltend Struktureinheiten der Formeln (I) und (II) 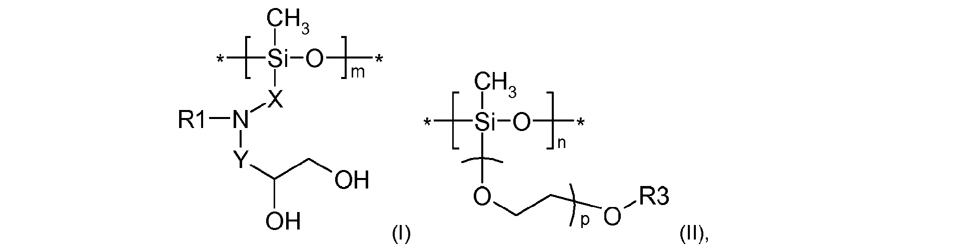 in denen X für einen substituierten oder unsubstituierten Alkylenrest mit 1, 2, 3, 4, 5, 6, 7 oder 8 C-Atomen steht, Y für einen substituierten oder unsubstituierten Alkylenrest mit 1, 2, 3, 4, 5, 6, 7 oder 8 C-Atomen steht, R1 für einen Methyl-, Ethyl-, n-Propyl-, Isopropyl-, n-Butyl-, sec-Butyl, Isobutyl- oder tert-Butylrest steht, R3 für -H, einen Methyl-, Ethyl-, n-Propyl-, Isopropyl-, n-Butyl-, sec-Butyl, Isobutyl- oder tert-Butylrest steht, p für eine ganze Zahl von 1 bis 30, vorzugsweise für 10, 11, 12, 13, 14, 15, 16, 17 oder 18 steht und m und n für ganze Zahlen zwischen 2 und 200 stehen.

## Beschreibung

Gegenstand der Erfindung ist ein Mittel zur Farbveränderung von keratinischen Fasern, welches in einem kosmetischen Träger neben mindestens einer farbgebenden Verbindung mindestens ein Silicon-Blockcopolymer enthält. Die erfindungsgemäßen Mittel eignen sich insbesondere zur Reduktion der Haarschädigung und zur Verbesserung der Haaroberfläche bei der oxidativen Färbung am menschlichen Haar. Darüber hinaus betrifft die Erfindung die Verwendung des Mittels zur Verringerung der Haarschädigung und zur Verbesserung der Haaroberfläche. Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Diese Mittel sollen neben der gewünschten Färbe- und Formleistung möglichst minimale Schädigungen auf dem Haar hervorrufen. Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme. Für permanente, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente so genannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Schließlich hat ein weiteres Färbeverfahren große Beachtung gefunden, bei dem Vorstufen des natürlichen Haarfarbstoffs Melanin auf das Substrat, z. B. Haare, aufgebracht werden, wobei diese dann im Rahmen oxidativer Prozesse im Haar naturanaloge Farbstoffe ausbilden. Oxidative Färbemittel enthalten in der Regel Wasserstoffperoxid als Oxidationsmittel. Dies führt neben dem gewünschten kosmetischen Effekt zu oxidativen Schädigungen der Haaroberfläche. Auch führt der üblicherweise in solchen Mitteln basisch eingestellte pH-Wert dazu, dass die Struktur des Haares oxidativ geschädigt wird. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids ist, desto stärkere Schädigungen werden in der Regel auf der Keratinfaser hervorgerufen. Insbesondere Spliss oder gar Bruch der Faser sind vom Verbraucher höchst unerwünschte Begleiterscheinungen einer Blondierung, die insbesondere bei mehrfacher Wiederholung des oxidativen Färbeprozesses auftreten können. Es ist daher besonders wünschenswert, Färbemittel bereitzustellen, die neben einer guten Färbeleistung einen positiven Beitrag zur Verbesserung der Faserstruktur leisten und damit gleichzeitig als Pflege- oder Konditioniermittel wirken. Somit könnte auf die sonst häufig erforderliche, zusätzliche pflegende Nachbehandlung verzichtet werden, die sowohl hinsichtlich Anwendungskomforts als zusätzlichen Behandlungsschritt als auch hinsichtlich Verpackungsökonomie mit Nachteilen behaftet ist. In der Vergangenheit erwiesen sich Versuche, handelsübliche Pflegestoffe in Färbemittel einzuarbeiten, häufig als unzureichend und wenig erfolgreich, da übliche Pflegestoffe für keratinische Fasern, insbesondere unter den harschen oxidativen Färbebedingungen, in Färbemittel häufig nur mangelnde Stabilität besitzen und dadurch nur eingeschränkt Wirkung zeigen. Es besteht somit weiterhin ein großer Verbesserungsbedarf für solche weniger schädigenden oder pflegenden Färbemittel. Weiterhin kann auch eine Verringerung der notwendigen Anwendungsmenge des Färbemittels zu einer Verbesserung der Faserbelastung führen. Es ist daher nicht nur aus ökonomischen Gesichtspunkten wünschenswert, Färbemittel mit einem verbesserten Färbevermögen, insbesondere einem verbesserten Farbaufzug auf die keratinische Faser, verbesserten Farberhalt und Homogenität der Färbung, bereitzustellen.

Aufgabe der vorliegenden Erfindung ist daher die Bereitstellung kosmetischer Mittel zur Farbveränderung keratinischer Fasern, die die oben beschriebenen Nachteile ausgleichen und die eine verringerte Haarschädigung aufweisen. Eine solche verringerte Haarschädigung lässt sich an der Haaroberfläche beispielsweise durch eine Verringerung von Haarbruch oder Spliss oder Verbesserung der Kämmbarkeit nachweisen.

In nicht vorhersehbarer Weise konnte nun gefunden werden, dass farbverändernde Mittel, die neben einer farbverändernden Komponente mindestens ein Silicon-Blockcopolymer enthalten, die oben genannten Nachteile vermeiden. Bedingt durch die pflegende Wirkung bei Anwendung des erfindungsgemäßen Mittels kann die Haarschädigung hierdurch deutlich verbessert werden.

Ein erster Gegenstand der Erfindung ist daher eine Zusammensetzung zur Farbveränderung keratinischer Fasern, enthaltend
a) mindestens eine Verbindung aus der Gruppe der Oxidationsfarbstoffvorprodukte, der direktziehenden Farbstoffe, Wasserstoffperoxid oder deren Mischungen
b) mindestens ein Silicon-Blockcopolymer, enthaltend Struktureinheiten der Formeln (I) und (II) 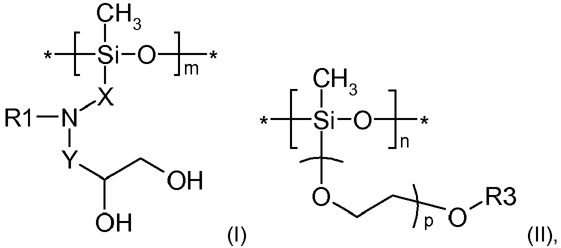
in denen
- X: für einen substituierten oder unsubstituierten Alkylenrest mit 1, 2, 3, 4, 5, 6, 7 oder 8 C-Atomen steht,
- Y: für einen substituierten oder unsubstituierten Alkylenrest mit 1, 2, 3, 4, 5, 6, 7 oder 8 C-Atomen steht,
- R1: für einen Methyl-, Ethyl-, n-Propyl-, Isopropyl-, n-Butyl-, sec-Butyl, Isobutyl- oder tert-Butylrest steht,
- R3: für -H, einen Methyl-, Ethyl-, n-Propyl-, Isopropyl-, n-Butyl-, sec-Butyl, Isobutyl- oder tert-Butylrest steht,
- p: für eine ganze Zahl von 1 bis 30, vorzugsweise für 10, 11, 12, 13, 14, 15, 16, 17 oder 18 steht
- m und n: für ganze Zahlen zwischen 2 und 200 stehen.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Erfindungsgemäße Mittel zum Färben enthalten mindestens einen direktziehende Farbstoff und/oder Oxidationsfarbstoffvorprodukte. Mittel, die gleichzeitig färbend und aufhellend wirken, enthalten meist sowohl Oxidationsmittel als auch mindestens einen direktziehende Farbstoff und/oder Oxidationsfarbstoffvorprodukte. Sind nur direktziehende Farbstoffe vorhanden, ist beispielsweise auch die Formulierung eines Färbeshampoos möglich.

Bevorzugte erfindungsgemäße Mittel sind Färbemittel, d.h. Mittel zur Veränderung der Farbe keratinischer Fasern. Unter diesen sind insbesondere die so genannten Oxidationsfärbemittel bevorzugt. Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten.

Erfindungsgemäß bevorzugte Mittel zum Färben und/oder Aufhellen keratinischer Fasern sind demnach dadurch gekennzeichnet, dass sie zusätzlich - bezogen auf ihr Gewicht - 0,001 bis 5 Gew.-% eines oder mehrerer Oxidationsfarbstoffvorprodukte und/oder direktziehender Farbstoffe enthalten. Diese werden weiter unten detailliert beschrieben.

Die erfindungsgemäßen Mittel enthalten als Pflegesubstanz zur Verhinderung oder zum Ausgleich von Feuchtigkeitsverlust und zur Verbesserung der mechanischen Eigenschaften, insbesondere zur Verhinderung oder zum Ausgleich von Sprödigkeit der keratinischen Fasern sowie zur Kopfhautschonung mindestens ein Silicon-Blockcopolymer, enthaltend Struktureinheiten der Formeln (I) und (II).

In der Formel für die Struktureinheit (I) stehen X und Y unabhängig voneinander für einen substituierten oder unsubstituierten Alkylenrest mit 1, 2, 3, 4, 5, 6, 7 oder 8 C-Atomen. Bevorzugte Reste X und Y sind unsubstituiert, d.h. es handelt sich vorzugsweise um reine Kohlenwasserstoffreste. Unter diesen sind die linearen Kohlenwasserstoffreste bevorzugt, d. h. bevorzugte Reste X und Y sind unabhängig voneinander ausgewählt aus -CH₂-, -(CH₂)₂-, -(CH₂)₃-, -(CH₂)₄-, -(CH₂)₅-, -(CH₂)₆-, -(CH₂)₇- oder -(CH₂)₈- Besonders bevorzugt steht X für eine der Gruppierungen -CH₂-, -(CH₂)₂- oder -(CH₂)₃- und Y für eine der Gruppierungen -CH₂-, -(CH₃)₂- oder -(CH₂)₃-, so dass bevorzugte erfindungsgemäße Zusammensetzungen mindestens ein Silicon-Blockcopolymer enthalten, das Struktureinheiten der Formel (I-1) bis (I-9) aufweist 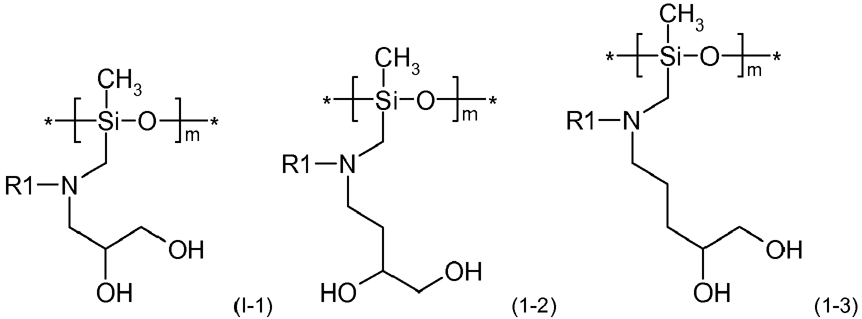 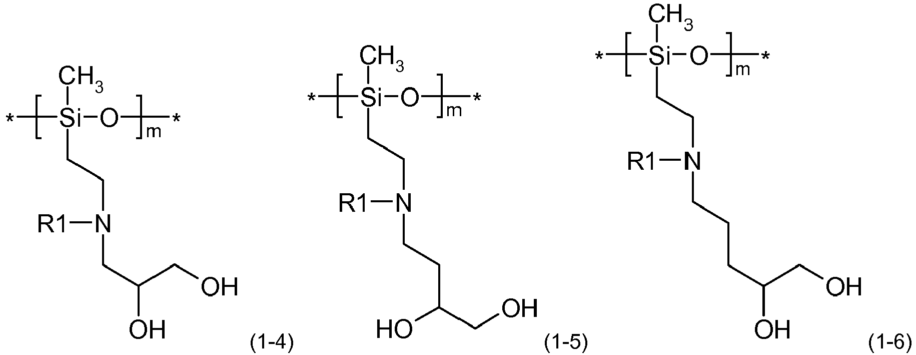 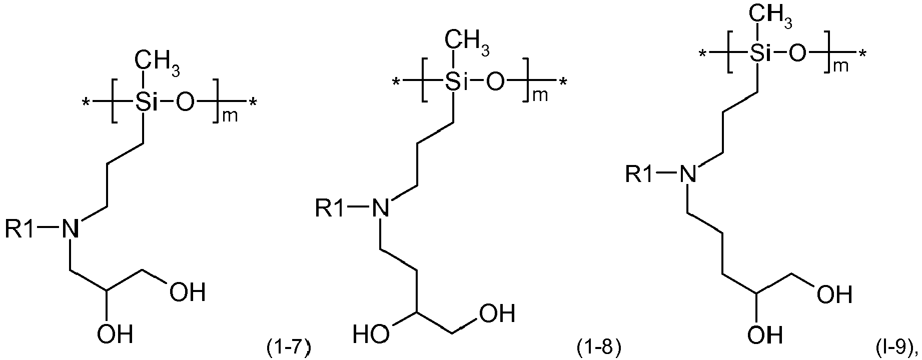 in denen
- R1: für einen substituierten oder unsubstituierten Methyl-, Ethyl-, n-Propyl-, Isopropyl-, n-Butyl-, sec-Butyl, Isobutyl- oder tert-Butylrest steht,
- m: für eine ganze Zahl zwischen 2 und 200 steht.

Ganz besonders bevorzugt gilt X = -(CH₂)₂- und Y = -CH₂-, so dass bevorzugte erfindungsgemäße Zusammensetzungen dadurch gekennzeichnet sind, dass sie mindestens ein Silicon-Blockcopolymer enthalten, das Struktureinheiten der Formel (I-4) enthalten.

Der Rest R1 steht für einen substituierten oder unsubstituierten Methyl-, Ethyl-, n-Propyl-, Isopropyl-, n-Butyl-, sec-Butyl, Isobutyl- oder tert-Butylrest. R1 ist vorzugsweise ausgewählt aus -CH₃, -CH₂CH₃, CH₂CH(CH₃)₂ und -CH₂OH, so dass bevorzugte erfindungsgemäße Zusammensetzungen mindestens ein Silicon-Blockcopolymer enthalten, das Struktureinheiten der Formel (I-10) bis (I-45) aufweist 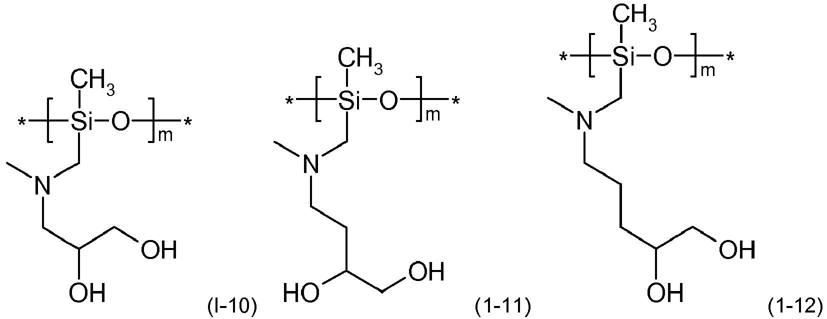 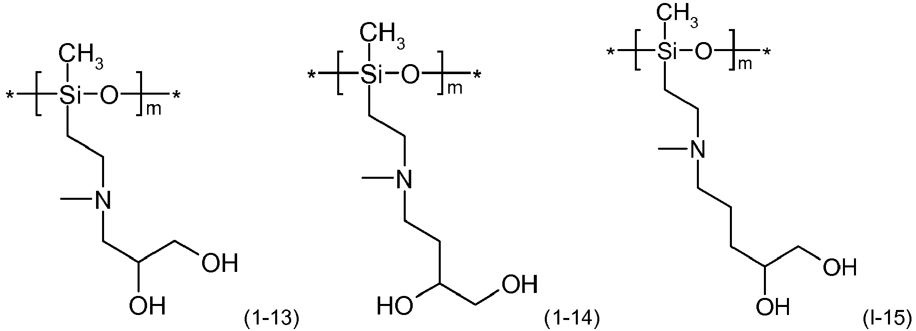 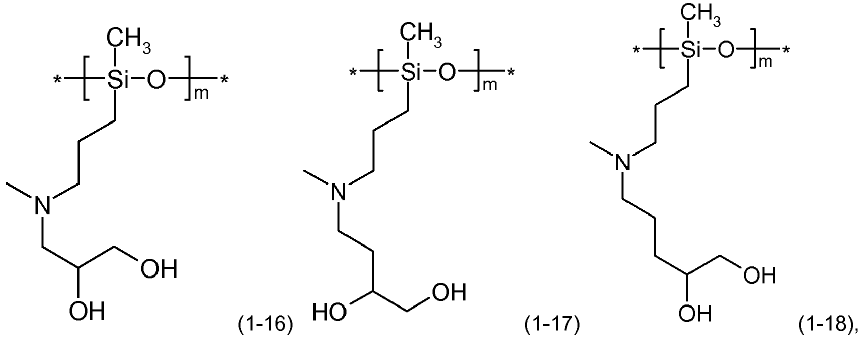 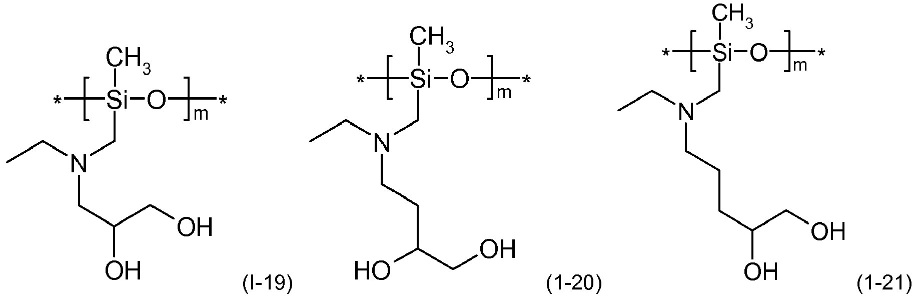 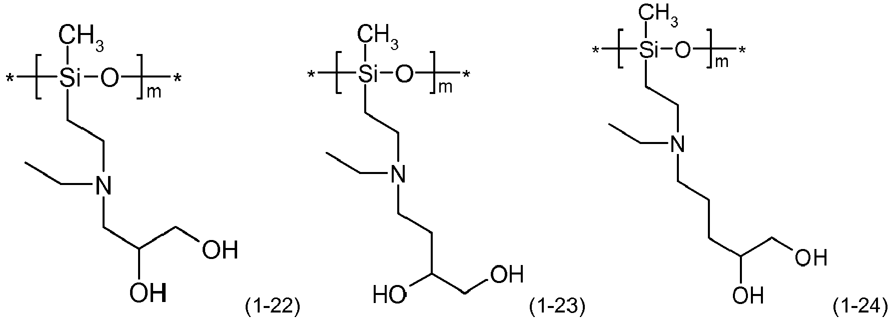 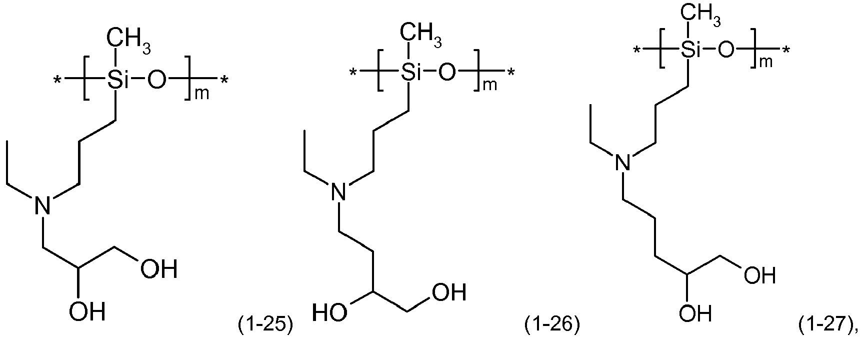 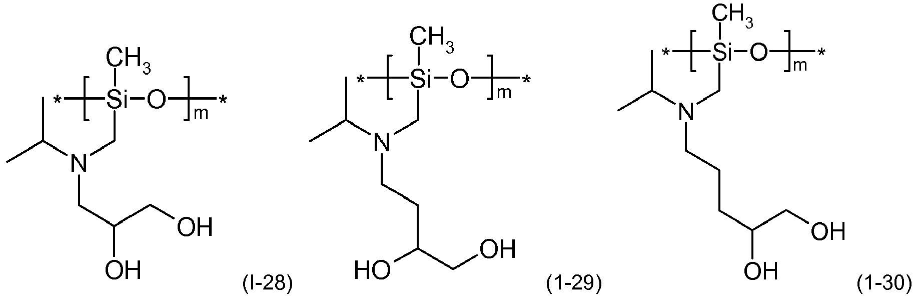 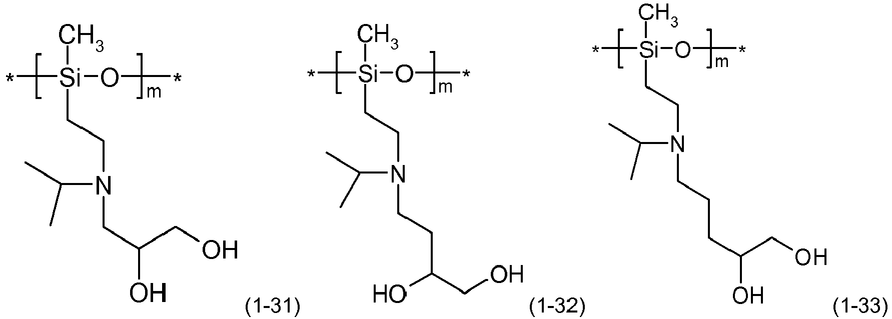 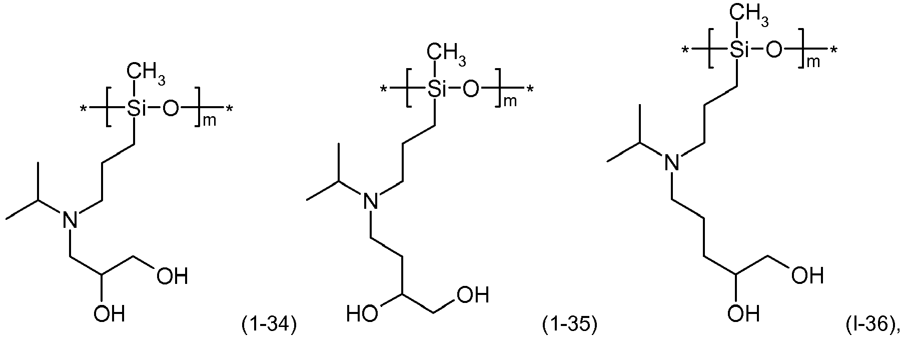 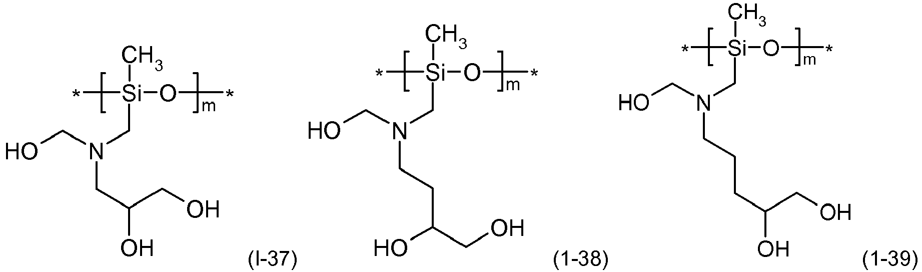 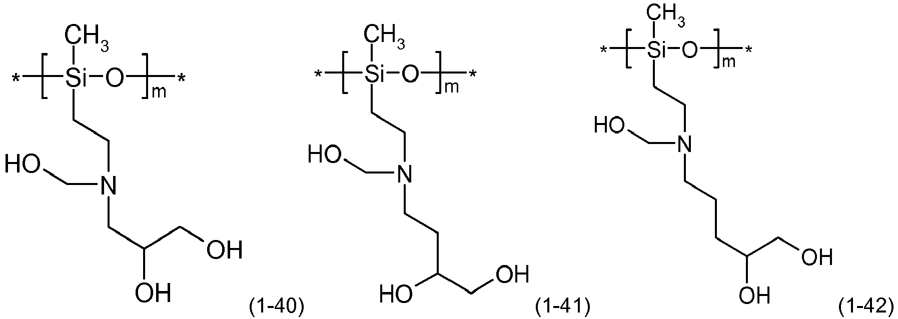 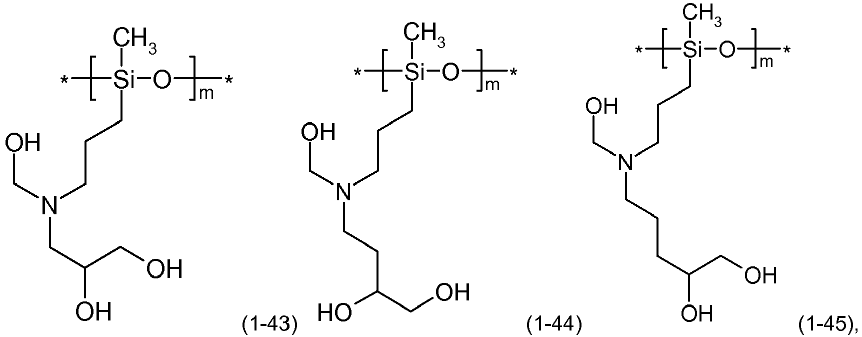 in denen m für eine ganze Zahl zwischen 2 und 200 steht.

Ganz besonders bevorzugt gilt R1 = -CH(CH₃)₂, so dass bevorzugte erfindungsgemäße Zusammensetzungen dadurch gekennzeichnet sind, dass sie mindestens ein Silicon-Blockcopolymer enthalten, das Struktureinheiten der Formel (la) 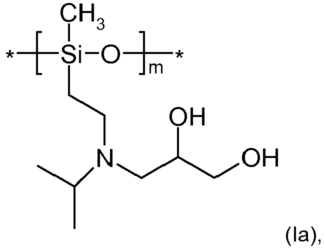 enthält, in der m für eine ganze Zahl zwischen 2 und 200 steht.

Die in den erfindungsgemäßen Mitteln enthaltenen Copolymere b) enthalten darüber hinaus Struktureinheiten der Formel (II) 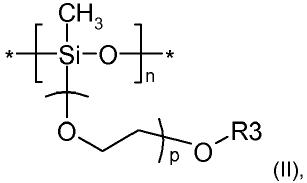 in der p für eine ganze Zahl von 1 bis 30, vorzugsweise für 10, 11, 12, 13, 14, 15, 16, 17 oder 18 steht und n für ganze Zahlen zwischen 2 und 200 steht.

R3 steht vorzugsweise für einen Methyl-, Ethyl, n-Propyl, Isopropyl- oder Isobutylrest, ganz besonders bevorzugt gilt R3 = -CH₂CH(CH₃)₂, so dass bevorzugte erfindungsgemäße Zusammensetzungen dadurch gekennzeichnet sind, dass sie mindestens ein Silicon-Blockcopolymer enthalten, das Struktureinheiten der Formel (IIa) 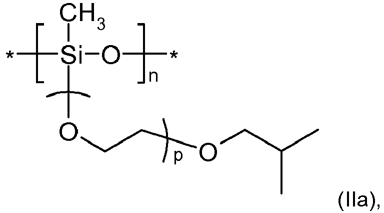 enthält, in der n für eine ganze Zahl zwischen 2 und 200 und p für die Zahl 10, 11, 12, 13, 14, 15 oder 16, vorzugsweise für 14, steht.

Die in den erfindungsgemäßen Mitteln enthaltenen Blockcopolymere b) können außer Blöcken aus Struktureinheiten der Formeln (I) und (II) weitere Strukturblöcke enthalten. Bevorzugt sind weitere Blöcke aus Polydimethylsiloxan, die die Blöcke aus Struktureinheiten der Formeln (I) und (II) terminieren oder voneinander trennen können.

Das bzw. die Copolymer(e) b) wird in den erfindungsgemäßen Mitteln vorzugsweise in Mengen bis zu 10 Gew.-%, jeweils bezogen auf die gesamte Zusammensetzung, eingesetzt. bevorzugte erfindungsgemäße Zusammensetzungen sind dadurch gekennzeichnet, dass sie - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% des/der Silicon-Blockcopolymer(e), enthaltend Struktureinheiten der Formeln (I) und (II), enthalten. Es hat sich gezeigt, dass ein Zusatz an Butyloctanol die Copolymer(e) b) in der erfindungsgemäßen Zusammensetzung stabilisieren kann, so dass die Pflegeffekte weiter verstärkt werden. Erfindungsgemäß bevorzugte Zusammensetzungen sind daher dadurch gekennzeichnet, dass sie - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% 2-Butyloctan-1-ol (INCI-Bezeichnung: Butyloctanol) enthalten.

Das/die Copolymer(e) b) wird/werden nach der Internationalen Nomenklatur für Kosmetik-Inhaltsstoffe (INCI) als Bis-Isopropylamino-PG-Propyl Dimethicone/Bis-Isobutyl PEG-14 Copolymer bezeichnet. Ein weiterer Gegenstand der vorliegenden Erfindung ist daher eine Zusammensetzung zur Farbveränderung keratinischer Fasern, enthaltend
a) mindestens eine Verbindung aus der Gruppe der Oxidationsfarbstoffvorprodukte, der direktziehenden Farbstoffe, Wasserstoffperoxid oder deren Mischungen
b) mindestens eine Verbindung mit der INCI-Bezeichnung Bis-Isopropylamino-PG-Propyl Dimethicone/Bis-Isobutyl PEG-14 Copolymer.

Die erfindungsgemäßen Mittel enthalten weiterhin eine farbgebende Verbindung, ausgewählt aus Oxidationsfarbstoffvorprodukten und/oder direktziehenden Farbstoffen und/oder Wasserstoffperoxid.

In einer bevorzugten Ausführungsform enthalten erfindungsgemäße Mittel als farbgebende Komponenten mindestens ein Oxidationsfarbstoffvorprodukt.

Die Oxidationsfarbstoffvorprodukte werden bevorzugt in einer Menge von 0,005 bis 25 Gew.-%, bevorzugt von 0,05 bis 20 Gew.-% und besonders bevorzugt von 0,1 bis 15 Gew.-%, jeweils bezogen auf das anwendungsbereite Oxidationsfärbemittel, verwendet.

In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate, einzusetzen.

Erfindungsgemäße Entwicklerkomponenten werden ausgewählt aus p-Phenylendiamin, zweikernigen Entwicklerkomponenten, p-Aminophenol, o-Aminophenol, heterocyclischen Entwicklerkomponenten und/oder den Derivaten vorstehender Substanzklassen. Die Entwicklerkomponenten werden bevorzugt in einer Menge von 0,05 bis 10 Gew.-%, vorzugsweise 0,1 bis 5 Gew.-%, jeweils bezogen auf das anwendungsbereite Oxidationsfärbemittel, verwendet.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylen-diamin, 2-Fluor-p-phenylendiamin, 2-Isopropyl-p-phenylendiamin, N-(2-Hydroxypropyl)-p-phenylen-diamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxy-ethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)-ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-[4-(methylamino)phenyl]tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxa-decan oder einem der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-[(2-hydroxyethyl)-aminomethyl]phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol oder einem der physiologisch verträglichen Salze dieser Verbindungen.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidinderivate sind die Verbindungen 2,4,5,6-Tetraaminopyrimidin, 4-Hydroxy-2,5,6-triaminopyrimidin, 2-Hydroxy-4,5,6-triamino-pyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxy-methylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze.

Bevorzugte Pyrazolopyrimidinderivate sind insbesondere die Derivate des Pyrazolo[1,5-a]pyrimidin und dessen tautomeren Formen, sofern ein tautomeres Gleichgewicht besteht. Die Pyrazolo[1,5-a]pyrimidinen sind insbesondere ausgewählt aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]-ethanol, 2-[(7-Aminopyrazolo[1,5-a]pyrimidin-3-yl)-(2-hydroxyethyl)amino]ethanol; 5,6-Dimethylpyr-azolo[1,5-a]pyrimidin-3,7-diamin, 2,6-Dimethylpyrazolo[1,5-a]pyrimidin-3,7-diamin, 3-Amino-7-di-methylamino-2,5-dimethylpyrazolo[1,5-a]pyrimidin sowie ihren physiologisch verträglichen Salzen und ihren tautomeren Formen, wenn ein tautomeres Gleichgewicht vorhanden ist.

Besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxy-ethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin,4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt ausgewählt aus m-Aminophenol, m-Diaminobenzol, o-Diaminobenzol, o-Aminophenol, Naphthalinderivaten mit mindestens einer Hydroxygruppe, Di- beziehungsweise Trihydroxybenzol, Pyridin, Pyrimidin, Monohydroxy- bzw. Monoaminoindol, Monohydroxy- bzw. Monoaminoindolin, Pyrazolon, Benzomorpholin, Chinoxalin und/oder den Derivaten der vorstehenden Substanzklassen. Die Kupplerkomponenten werden bevorzugt in einer Menge von 0,005 bis 10 Gew.-%, vorzugsweise 0,1 bis 5 Gew.-%, jeweils bezogen auf das anwendungsbereite Färbemittel, verwendet.

Die erfindungsgemäß bevorzugten m-Aminophenole bzw. deren Derivate werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-amino-phenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-methylamino-benzol, 3-Ethylamino-4-methylphenol, 2,4-Dichlor-3-aminophenol und den physiologisch verträglichen Salzen der vorstehend genannten Verbindungen.

Die erfindungsgemäß bevorzugten m-Diaminobenzole bzw. deren Derivate werden ausgewählt aus mindestens einer Verbindung aus der Gruppe, die gebildet wird aus m-Phenylendiamin, 2-(2,4-Di-aminophenoxy)ethanol, 1,3-Bis(2,4-diaminophenoxy)propan, 1-Methoxy-2-amino-4-(2'-hydroxy-ethylamino)benzol, 1,3-Bis(2,4-diaminophenyl)propan, 2,6-Bis(2'-hydroxyethylamino)-1-methyl-benzol, 2-({3-[(2-Hydroxyethyl)amino]-4-methoxy-5-methylphenyl}amino)ethanol, 2-({3-[(2-Hydroxy-ethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)aminobenzol und den physiologisch verträglichen Salzen der genannten Verbindungen.

Die erfindungsgemäß bevorzugten o-Diaminobenzole bzw. deren Derivate werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen der genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß bevorzugten Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxy-naphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß bevorzugten Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß bevorzugten Indolinderivate werden ausgewählt aus mindestens einer Verbindung der Gruppe, die gebildet wird aus 4-Hydroxyindolin, 6-Hydroxyindolin und 7-Hydroxy-indolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Amino-phenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diamino-phenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methyl-phenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxy-benzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxy-naphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Oxidationsfarbstoffvorprodukte vom Entwicklertyp und vom Kupplertyp werden besonders bevorzugt in bestimmten Kombinationen eingesetzt. Mit den als Kombination genannten Oxidationsfarbstoffvorprodukten und/oder deren physiologisch verträglichen Salzen können jedoch auch noch weitere Farbstoffvorprodukte kombiniert werden. Bevorzugte Mittel enthalten daher als Oxidationsfarbstoffprodukt zumindest eine der Kombinationen, ausgewählt aus p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)-benzol; p-Toluylendiamin / 2-Amino-3-hydroxypyridin; p-Toluylendiamin / 1-Naphthol; 2-(2-Hydroxy-ethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis-(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylen-diamin / 2-Amino-3-hydroxypyridin; 2-(2-Hydroxyethyl)-p-phenylendiamin / 1-Naphthol; 2-Methoxy-methyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxy-methyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methyl-phenyl)-N-[3-(1H-imidazol-1-yl)propyl]amin / Resorcin; N-(4-Amino-3-methylphenyl)-N-[3-(1H-imid-azol-1-yl)propyl]amin / 2-Methylresorcin; N-(4-Amino-3-methylphenyl)-N-[3-(1H-imidazol-1-yl)pro-pyl]amin / 5-Amino-2-methylphenol; N-(4-Amino-3-methylphenyl)-N-[3-(1H-imidazol-1-yl)propyl]-amin / 3-Aminophenol; N-(4-Amino-3-methylphenyl)-N-[3-(1H-imidazol-1-yl)propyl]amin / 2-(2,4-Diaminophenoxy)ethanol; N-(4-Amino-3-methylphenyl)-N-[3-(1H-imidazol-1-yl)propyl]amin / 1,3-Bis(2,4-diaminophenoxy)propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxy-ethyl)pyrazol / 2-(2,4-Diaminophenoxy)ethanol; 4,5-Diamino-1-(2-hydroxyethyl)pyrazol/1,3-Bis(2,4-diaminophenoxy)propan; 4,5-Diamino-1-(2-hydroxyethyl)pyrazol/1-Methoxy-2-amino-4-(2-hydroxy-ethylamino)benzol; 4,5-Diamino-1-(2-hydroxyethyl)pyrazol/2-Amino-3-hydroxypyridin sowie 4,5-Diamino-1-(2-hydroxyethyl)pyrazol/1-Naphthol.

Besonders bevorzugte erfindungsgemäße Zusammensetzung sind dadurch gekennzeichnet, dass sie als Oxidationsfarbstoffvorprodukt mindestens eine Entwicklerkomponente und/oder mindestens eine Kupplerkomponente enthalten, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'- diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6- triaminopyrimidin, 4,5-Diamino-1-(2-hydroxyethyl)-pyrazol und bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3-Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

Um eine ausgewogene und subtile Nuancenausbildung zu erzielen, ist es erfindungsgemäß vorteilhaft, wenn die erfindungsgemäßen Mittel weitere farbgebende Komponenten enthalten.

Weiterhin können die erfindungsgemäßen Mittel als farbgebende Komponente, insbesondere zur Nuancierung, mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 5 Gew.-%, bezogen auf das gesamte Mittel, eingesetzt. Die Gesamtmenge an direktziehenden Farbstoffen beträgt vorzugsweise höchstens 3 Gew.-%. Direktziehende Farbstoffe sind als anionische, kationische und nichtionische direktziehende Farbstoffe bekannt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Blue 347, Basic Brown 16 und Basic Brown 17, kationische Anthrachinonfarbstoffe wie HC Blue 16 (Bluequat B), sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Besonders bevorzugte erfindungsgemäße Zusammensetzung sind dadurch gekennzeichnet, dass sie mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxy-ethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitro-benzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

In einer weiteren Ausführungsform der vorliegenden Erfindung wird als farbgebende Komponente mindestens eine Farbstoffvorstufe naturanaloger Farbstoffe zugegeben.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, insbesondere 0,1 bis 3,5 Gew.-%, bezogen auf das Gesamtgewicht der Mittel, eingesetzt.

Bevorzugte Derivate des Indolins sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure und besonders bevorzugt 5,6-Dihydroxyindolin. Bevorzugte Derivate des Indols sind 5,6-Dihydroxyindol, N-Methyl-5,6-dihydroxyindol, N-Ethyl-5,6-dihydroxy-indol, N-Propyl-5,6-dihydroxyindol, N-Butyl-5,6-dihydroxyindol, 5,6-Dihydroxyindol-2-carbonsäure sowie insbesondere 5,6-Dihydroxyindol.

Die erfindungsgemäßen Mittel können auch als Aufhellmittel (nachfolgend auch als Blondiermittel bezeichnet) und/oder als Färbemittel bereitgestellt werden. Mittel, welche gleichzeitig färbend und aufhellend wirken, werden auch als aufhellende Färbemittel bezeichnet.

Aufhellende erfindungsgemäße Mittel enthalten mindestens ein Oxidationsmittel, ausgewählt aus Wasserstoffperoxid und dessen Anlagerungsverbindungen an feste Träger. Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon n H₂O₂ (n ist eine positive ganze Zahl größer 0), Harnstoffperoxid und Melaminperoxid, eingesetzt.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozen-tige Lösungen in Wasser verwendet. Erfindungsgemäß bevorzugte Mittel zum Färben und/oder Aufhellen keratinischer Fasern sind dadurch gekennzeichnet, dass sie 0,5 bis 15 Gew.-%, vorzugsweise 1 bis 12,5 Gew.-%, besonders bevorzugt 1 bis 10 Gew.-% und insbesondere 1 bis 8 Gew.-% Wasserstoffperoxid (berechnet als 100%iges H₂O₂) enthalten.

Besonders bevorzugte erfindungsgemäße Zusammensetzungen sind dadurch gekennzeichnet, dass sie als Blondiermittel oder Entwickler konfektioniert sind und - bezogen auf sein Gewicht - 0,5 bis 15 Gew.-%, vorzugsweise 0,75 bis 10 Gew.-%, besonders bevorzugt 1 bis 7,5 Gew.-%, weiter bevorzugt 1,25 bis 7 Gew.-% und insbesondere 1,5 bis 6,0 Gew.-% Wasserstoffperoxid (berechnet als 100 %iges H₂O₂) enthalten.

Bevorzugt werden die erfindungsgemäßen Mittel als fließfähige Zubereitungen formuliert. Dabei sollten die Mittel so formuliert werden, dass das Mittel sich einerseits gut am Anwendungsort auftragen und verteilen lässt, andererseits jedoch ausreichend viskos ist, dass das Mittel während der Einwirkzeit am Wirkort verbleibt und nicht verläuft.

Es hat sich daher erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Bevorzugt handelt es sich bei dem Verdickungsmittel um ein anionisches, synthetisches Polymer. Bevorzugte anionische Polymere sind Acrylsäure- und/oder Methacrylsäure-Polymere oder -Copolymerisate, die in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogenen Monomereinheiten, bevorzugt Acrylamid, Methacrylamid, Acrylsäure-C₁-C₄-Alkylester und Methacrylsäure-C₁-C₄-Alkylester, eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der Erfindung erwiesen. Insbesondere Tone, vorzugsweise Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.

Besonders vorteilhafte Verdickungsmittel sind dabei solche, die neben der Verdickungsleistung zusätzlich einen Beitrag zu den gewünschten Eigenschaften der Mittel, wie beispielsweise der Pflegevermögen, leisten. Es zeigte sich, dass gerade amphotere Polymere diese Eigenschaft in besonderem Maße aufweisen. In einer bevorzugten Ausführungsform enthalten die erfindungsgemäßen Mittel jedoch zusätzlich mindestens ein amphoteres Polymer. Dabei kann das amphotere Polymer durch Polymerisation ethylenischer Monomere aufgebaut sein, wobei sowohl aus verschiedenen, kationische Gruppen tragenden Monomeren sowie verschiedenen, anionische Gruppen tragenden Monomeren und gegebenenfalls auch nichtionische Monomeren eingesetzt werden können. Bevorzugte kationische Monomere sind dabei Dialkyldiallylammonium-Verbindungen, quaternäre Alkyl-Vinyl-Imidazolium-Verbindungen sowie (Meth-)Acrylsäureester oder (Meth-)Acrylsäureamide mit Trialkylammonioalkyl-Resten. Bevorzugte anionische Monomere sind dabei physiologisch verträgliche Salze der Acrylsäure oder Methacrylsäure. Geeignete, gegebenenfalls zusätzlich einzusetzende nichtionische Monomere sind Vinylcaprolactam, Vinylpyrrolidinon, Vinylimidazol, Acrylamid und Methacrylamid.

In einer bevorzugten Ausführungsform des ersten Erfindungsgegenstands enthält das Mittel zusätzlich mindestens ein amphoteres Polymer, welches sich ableitet von mindestens einem kationi-schen Monomer mit quartären Ammoniumgruppen der allgemeinen Formel (I), 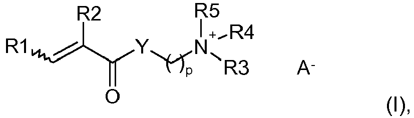 in der R1 und R2 unabhängig voneinander für Wasserstoff oder eine Methylgruppe stehen, R3, R4 und R5 unabhängig voneinander für Alkylgruppen mit 1 bis 4 Kohlenstoff-Atomen stehen, Y eine NH-Gruppe oder ein Sauerstoffatom, p eine ganze Zahl von 2 bis 5 und A⁻ ein physiologisch verträgliches Anion einer organischen oder anorganischen Säure ist,
und von mindestens einem anionischen Monomer, ausgewählt aus Acrylsäure und/oder Methacrylsäure und/oder einem deren physiologisch verträglichen Salze.

Darin steht besonders bevorzugt in Formel (I) die Gruppe Y für NH. p steht bevorzugt für die Zahl 3. R1 und R2 stehen bevorzugt für Wasserstoff. R3, R4 und R5 stehen bevorzugt für Methyl. A⁻ steht bevorzugt für Chlorid.

Besonders bevorzugte amphotere Polymere werden unter der INCI-Bezeichnung "Acrylamidopropyltrimonium Chloride/Acrylates Copolymer" vertrieben.

Die amphoteren Polymere werden in bevorzugt in einer Gesamtmenge von 0,01 bis 5 Gew.%, bevorzugt 0,05 bis 3 Gew.% und ganz besonders bevorzugt von 0,1 bis 2 Gew.%, bezogen auf die Gesamtmenge des anwendungsbereiten Mittels, eingesetzt.

Üblicherweise enthalten die erfindungsgemäßen Mittel zusätzlich als oberflächenaktive Substanz ein Emulgator bzw. ein Tensid, wobei oberflächenaktive Substanzen je nach Anwendungsgebiet als Tenside oder als Emulgatoren bezeichnet werden. Bevorzugt sind dabei insbesondere anionischen und nichtionischen Tenside und Emulgatoren.

Eine weitere Ausführungsform der vorliegenden Erfindung ist daher dadurch gekennzeichnet, dass das Mittel zusätzlich mindestens ein nichtionisches und/oder anionisches Tensid enthält.

Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Alkylsulfate, Alkylethersulfate, jeweils mit 10 bis 18 C-Atomen in der Alkylgruppe, und polyalkoxylierte Ethercarbonsäuren mit 6 bis 20 C-Atomen in der Alkylgruppe und bis zu 12 Glykolethergruppen im Molekül.

Eine weitere Ausführungsform des ersten Erfindungsgegenstands ist dabei dadurch gekennzeichnet, dass das Mittel als zusätzliches anionisches Tensid mindestens eine Ethercarbonsäure der Formel RO(CH₂CH₂O)ₓCH₂COOH und/oder eines ihrer physiologisch verträglichen Salze enthält, worin R für eine C₆-C₂₀-Alkylkette steht und x = 0 oder eine Zahl 1 bis 16 ist.

Beispiele bevorzugter Verbindungen vom Typ der polyalkoxylierten Ethercarbonsäuren werden unter den Handelsnamen Akypo TEC (INCI-Bezeichnung: Oleth-10 Carboxylic Acid, Capryleth-9 Carboxylic Acid, Hexeth-4 Carboxylic Acid), Akypo RLM 38 (INCI-Bezeichnung: Laureth-5 Carboxylic Acid), Akypo RO 90 (INCI-Bezeichnung: Oleth-10 Carboxylic Acid), Akypo Soft 100 (INCI-Bezeichnung: Sodium Laureth-11 Carboxylate), Akypo RO 20 (INCI-Bezeichnung: Oleth-3 Carboxylic Acid), Akypo Soft 45 HP (INCI-Bezeichnung: Sodium Laureth-6 Carboxylate) oder Akypo RLM 45 (INCI-Bezeichnung: Laureth-6 Carboxylic Acid) vertrieben.

Die anionischen Tenside werden in bevorzugt in einer Gesamtmenge von 0,1 bis 30 Gew.%, bevorzugt 1 bis 25 Gew.% und ganz besonders bevorzugt von 1 bis 15 Gew.%, bezogen auf die Gesamtmenge des anwendungsbereiten Mittels, eingesetzt.

Als besonders vorteilhaft haben sich außerdem oberflächenaktive Verbindungen vom Typ nichtionischer Tenside herausgestellt. Solche Verbindungen sind beispielsweise Anlagerungsprodukte von Polyethylenoxid an Fettalkohole, wobei Anlagerungsprodukte von Polyethylenoxid an Fettalkohole besonders bevorzugt sind, die einen durchschnittlichen Ethoxylierungsgrad von 10 bis 45, insbesondere von 12 bis 30 aufweisen, wie beispielsweise Steareth-20, Coceth-15, Oleth-20 oder auch Ceteareth-30, sowie Alkylpolyglucoside entsprechend der allgemeinen Formel RO-(Z)ₙ, wobei R für Alkyl, Z für Zucker sowie n für die Anzahl der Zuckereinheiten steht.

Eine weitere Ausführungsform des ersten Erfindungsgegenstands ist dabei dadurch gekennzeichnet, dass das Mittel als zusätzliches nichtionisches Tensid mindestens ein Alkylpolyglucosid der Formel R'O-(Z)ₙ, worin R' für eine C₁₀-C₂₀-Alkylkette, Z für einen Zuckerrest mit 5 oder 6 Kohlenstoffatomen und n für eine Zahl von 1 bis 15 steht.

Beispiele solcher Verbindungen tragen die INCI-Bezeichnungen Decyl Glucoside, Lauryl Glucoside, Cetearyl Glucoside und Coco-Glucoside.

Erfindungsgemäß bevorzugte Mittel enthalten als nichtionisches Tensid das Anlagerungsprodukt von Cocos-Fettalkohol an (Poly-)glucose, welches unter der INCI-Bezeichnung Coco-Glucoside bekannt ist. Ein solches Produkt wird unter anderem unter dem Handelsnamen Lamesoft PO 65 (INCI-Bezeichnung: Coco-Glucoside, Glyceryl Oleate, Aqua) vertrieben.

Die nichtionischen Tenside werden in bevorzugt in einer Gesamtmenge von 0,1 bis 30 Gew.%, bevorzugt 1 bis 20 Gew.% und ganz besonders bevorzugt von 1 bis 10 Gew.%, bezogen auf die Gesamtmenge des anwendungsbereiten Mittels, eingesetzt.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Eine weitere Ausführungsform der Erfindung ist daher ein Mittel, welches zusätzlich ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die Anlagerungsprodukte an Harnstoff, Melamin, Polyvinylpyrrolidinon sowie Natriumborat in Frage. Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel.

Bei einer Anwendung von zusätzlichen Oxidationsmitteln wird das eigentliche Färbemittel zweckmäßigerweise unmittelbar vor der Anwendung durch Mischung einer erfindungsgemäßen Zubereitung, enthaltend in einem kosmetischen Träger mindestens eine farbgebende Verbindung, sowie einer Zubereitung, enthaltend das zusätzliche Oxidationsmittel, insbesondere Wasserstoffperoxid, hergestellt. Das erfindungsgemäße Mittel kann dabei das mindestens eine Silicon-Blockcopolymer, enthaltend Struktureinheiten der Formeln (I) und (II) gemäß den Patentansprüchen in der Zubereitung mit den farbgebenden Verbindungen und/oder in der Oxidationsmittelzubereitung enthalten. Bevorzugt enthält die Zubereitung mit den farbgebenden Verbindungen das mindestens eine Silicon-Blockcopolymer, enthaltend Struktureinheiten der Formeln (I) und (II) gemäß den Patentansprüchen.

Die vorgenannte Oxidationsmittelzusammensetzung der erfindungsgemäßen Zusammensetzungen weist einen pH-Wert im Bereich von 2 bis 6, bevorzugt im Bereich von 3 bis 5,5, auf, um das Oxidationsmittel stabil lagern zu können. Damit man nach dem Vermischen der Oxidationsmittelzusammensetzung mit der farbgebenden Zusammensetzung ein anwendungsbereites Mittel mit einer ausreichend hohen Viskosität erhält, die sich gut auf das Haar auftragen lässt und nicht vom Haar abtropft, enthält einer weiteren bevorzugten Ausführungsform die Oxidationsmittelzusammensetzung zusätzlich mindestens ein anionisches polymeres Verdickungsmittel, ausgewählt aus Homo- oder Copolymeren von Acrylsäure und/oder Methacrylsäure.

Da die Oxidationsmittelzusammensetzung einen sauren pH-Wert besitzt, die Anwendungsmischung jedoch einen alkalischen pH-Wert aufweist, unterliegt das polymere Verdickungsmittel einer pH-Wertveränderung, durch die die Carbonsäuregruppen von Acrylsäure- oder Methacrylsäure-Einheiten deprotoniert werden, was eine Gelbildung und damit eine Viskositätserhöhung bewirkt.

Neben Acrylsäure und Methacrylsäure sind Crotonsäure, Itaconsäure, Maleinsäureanhydrid und 2-Acrylamido-2-methylpropansulfonsäure weitere Beispiele für anionische Monomere, aus denen die polymeren anionischen Verdickungsmittel bestehen können. Dabei können die sauren Gruppen auch als Natrium-, Kalium-, Ammonium-, Mono- oder Triethanolammonium-Salz vorliegen. Bevorzugte anionische Homopolymere sind unvernetzte und vernetzte Polyacrylsäuren; besonders bevorzugt sind vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Innerhalb dieser Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidinon, Vinylether und Vinylester. Bevorzugt kann die erfindungsgemäße Oxidationsmittelzubereitung zusätzlich mindestens ein anionisches Acrylsäure- und/oder Methacrylsäure-Polymerisat oder -Copolymerisat enthalten. Bevorzugte Polymerisate dieser Art sind (Gew.-%-Angaben beziehen sich auf das Gewicht des Polymers):
- vernetzte Polymerisate aus 10 bis 60 Gew.-% Acrylsäure-C1-C4-Alkylester, 25 bis 70 Gew.-% Methacrylsäure und ggf. bis zu 40 Gew.-% eines weiteren Comonomeren,
- vernetzte Mischpolymerisate aus 50 bis 75 Gew.-% Ethylacrylat, 25 bis 35 Gew.-% Acrylsäure und 0 bis 25 Gew.-% anderer Comonomeren, als Dispersion im Handel erhältlich, z.B. unter der Handelsbezeichnung Latekoll^{®} D (BASF),
- Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Bevorzugt sind aber auch unvernetzte Copolymere aus mindestens einem Monomer, ausgewählt aus Acrylsäure, Methacrylsäure, Acrylsäure-C₁-C₆-Alkylestern und Methacrylsäure-C₁-C₆-Alkyl-estern, und mindestens einem Monomer, ausgewählt aus den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige unvernetzte Copolymere, die auch als Assoziativ-verdicker bezeichnet werden, werden beispielsweise von der Firma Rohm & Haas unter der Handelsbezeichnung Aculyn^{®} 22 sowie von der Firma Akzo Nobel unter den Handelsbezeichnungen Structure^{®}2001 und Structure^{®} 3001 vertrieben.

Besonders bevorzugte vernetzte anionische Copolymere sind beispielsweise Copolymere aus Acrylsäure, Methacrylsäure, Acrylsäure-C₁-C₆-Alkylestern oder Methacrylsäure-C₁-C₆-Alkylestern, wie sie unter der INCI-Deklaration Acrylates Copolymers vertrieben werden. Bevorzugt ist dabei die Kombination aus Methacrylsäure und Ethylacrylat mit vernetzenden, multifunktionalen Monomeren. Ein bevorzugtes Handelsprodukt dafür ist beispielsweise Aculyn^{®} 33 bzw. Aculyn^{®} 33A der Firma Rohm & Haas.

Bevorzugte erfindungsgemäße Oxidationsmittelzusammensetzungen (= Entwickler) oder bevorzugte erfindungsgemäße Mehrkomponentenverpackungseinheit (Kit-of-Parts), sind dadurch gekennzeichnet, dass mindestens ein anionisches Acrylsäure- und/oder Methacrylsäure-Polymerisat oder -Copolymerisat in einer Gesamtmenge von 0,1 bis 5 Gew.-%, besonders bevorzugt von 1 bis 4 Gew.-% und insbesondere von 2 bis 3 Gew.-%, jeweils bezogen auf das Gewicht der Oxidationsmittelzusammensetzung (II), enthalten ist.

Weitere bevorzugte erfindungsgemäße Zusammensetzungen sind als Blondiermittel oder Entwickler konfektioniert ist und enthalten, jeweils bezogen auf ihr Gewicht, 0,5 bis 15 Gew.-%, vorzugsweise 0,75 bis 10 Gew.-%, besonders bevorzugt 1 bis 7,5 Gew.-%, weiter bevorzugt 1,25 bis 7 Gew.-% und insbesondere 1,5 bis 6,0 Gew.-% Wasserstoffperoxid (berechnet als 100 %iges H₂O₂) und zusätzlich mindestens ein anionisches Acrylsäure- und/oder Methacrylsäure-Polymerisat oder -Copolymerisat in einer Gesamtmenge von 0,1 bis 5 Gew.-%, besonders bevorzugt von 1 bis 4 Gew.-% und insbesondere von 2 bis 3 Gew.-%.

Weiterhin hat es sich als vorteilhaft erweisen, wenn die Mittel, gegebenenfalls auch Zubereitung, enthaltend farbgebende Verbindungen, und/oder die Oxidationsmittelzubereitungen von oxidativen Färbemitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS), Diethylentriaminpentaessigsäure und ihre Salze, insbesondere Pentasodium Pentetate, und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta-(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Die erfindungsgemäßen Mittel enthalten weitere Hilfs- und Zusatzstoffe. Bevorzugt enthalten die fließfähigen Zubereitungen zusätzlich als weitere oberflächenaktive Substanz ein kationisches, amphoteres und/oder zwitterionisches Tensid.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Ein bevorzugtes zwitterionisches Tensid ist das unter der INCI-Bezeichnung Cocamidopropyl Betaine bekannte Fettsäureamid-Derivat.

In einer weiteren, bevorzugten Ausführungsform der vorliegenden Erfindung enthält das Mittel weiterhin mindestens ein amphoteres Tensid. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (BASF), Miranol C2M (Rhodia) und Ampholak XCO 30 (Akzo Nobel) vermarktet.

Die zwitterionischen und amphoteren Tenside sind bevorzugt in einer Gesamtmenge von 0,1 - 15 Gew.-%, bevorzugt 0,5 - 10 Gew.-% und ganz besonders bevorzugt von 1 - 5 Gew.-%, bezogen auf die Gesamtmenge des anwendungsbereiten Mittels, enthalten.

Erfindungsgemäß bevorzugt sind ebenfalls kationische Tenside vom Typ der quartären Ammoniumverbindungen, der Esterquats und der Amidoamine. Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weiterhin wurde überraschend festgestellt, dass die konditionierende Wirkung, die Verringerung der Haarschädigung und/oder der erzielte Färbeergebnis der erfindungsgemäßen Zusammensetzungen weiter verbessert werden kann, wenn zusätzlich mindestens ein Fett und/oder mindestens ein Öl in einer Gesamtmenge von 0,1 bis 90 Gew.-%, bevorzugt 1,0 bis 80 Gew.-%, besonders bevorzugt 5,0 bis 60 Gew.-%, außerordentlich bevorzugt 10 bis 45 Gew.-%, jeweils bezogen auf das Gewicht der Zusammensetzung, enthalten ist. Besonders bevorzugt ist es, wenn die Oxidationsmittel-freie erfindungsgemäße haarfarbverändernde Zusammensetzung mindestens ein Fett und/oder mindestens ein Öl in einer Gesamtmenge von 0,1 bis 90 Gew.-%, bevorzugt 1,0 bis 80 Gew.-%, besonders bevorzugt 5,0 bis 60 Gew.-%, außerordentlich bevorzugt 10 bis 45 Gew.-%, jeweils bezogen auf ihr Gewicht, enthält. In einer anderen bevorzugten Ausführungsform enthält die Oxidationsmittelzusammensetzung mindestens ein Fett und/oder mindestens ein Öl in einer Gesamtmenge von 0,1 bis 90 Gew.-%, bevorzugt 1,0 bis 80 Gew.-%, besonders bevorzugt 5,0 bis 60 Gew.-%, außerordentlich bevorzugt 10 bis 45 Gew.-%, jeweils bezogen auf ihr Gewicht. In einer dritten, erfindungsgemäß ebenfalls bevorzugten Ausführungsform enthält das anwendungsbereite haarfarbverändernde Mittel, also die Mischung aus haarfarbverändernder Zusammensetzung und Oxidationsmittelzusammensetzung, mindestens ein Fett und/oder mindestens ein Öl in einer Gesamtmenge von 0,1 bis 90 Gew.-%, bevorzugt 1,0 bis 80 Gew.-%, besonders bevorzugt 5,0 bis 60 Gew.-%, außerordentlich bevorzugt 10 bis 45 Gew.-%, jeweils bezogen auf das Gewicht des anwendungsbereiten haarfarbverändernden Mittels. In einer weiteren erfindungsgemäß ebenfalls bevorzugten Ausführungsform ist das mindestens eine Silicon-Blockcopolymer, enthaltend Struktureinheiten der Formeln (I) und (II) gemäß den Ansprüchen 1, 2, 3, 4, 5 oder 6, in der Oxidationsmittel-freien haarfarbverändernden Zusammensetzung und weiterhin mindestens ein Fett und/oder mindestens ein Öl in einer Gesamtmenge von 0,1 bis 90 Gew.-%, bevorzugt 1,0 bis 80 Gew.-%, besonders bevorzugt 5,0 bis 60 Gew.-%, außerordentlich bevorzugt 10 bis 45 Gew.-%, jeweils bezogen auf das Gewicht der Oxidationsmittel-freien haarfarbverändernden Zusammensetzung, enthalten. Der Begriff Öl meint "kosmetisches" Öl, also Öle, die zur Anwendung in Kosmetika geeignet und zugelassen sind. Erfindungsgemäß bevorzugte kosmetische Öle sind ausgewählt aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan (erhältlich z. B. unter dem Handelsnamen Cetiol^{®}S von BASF). Weitere erfindungsgemäß bevorzugte kosmetische Öle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldodecylester, z. B. erhältlich als Handelsprodukt Finsolv^{®} BOD. Weitere erfindungsgemäß bevorzugte kosmetische Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol^{®} G 16), 2-Octyldodecanol (Eutanol^{®} G), 2-Ethylhexylalkohol und Isostearylalkohol. Weitere bevorzugte kosmetische Öle sind ausgewählt aus Mischungen aus Guerbetalkoholen und Guerbetalkoholestern, z.B. dem Handelsprodukt Cetiol^{®} PGL (2-Hexyldecanol und 2-Hexyldecyllaurat). Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C₈₋₃₀-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol^{®} 318, Myritol^{®} 331 (BASF) oder Miglyol^{®} 812 (Hüls) mit unverzweigten Fettsäureresten sowie Glyceryltriisostearin mit verzweigten Fettsäureresten. Weitere erfindungsgemäß besonders bevorzugte kosmetische Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat. Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Hexyldecylstearat (Eutanol^{®} G 16 S), 2-Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat. Weitere erfindungsgemäß bevorzugte kosmetische Öle sind ausgewählt aus den Anlagerungsprodukten von 1 bis 5 Propylenoxid-Einheiten an ein- oder mehrwertige C₈₋₂₂-Alkanole, wie Octanol, Decanol, Decandiol, Laurylalkohol, Myristylalkohol und Stearylalkohol, z. B. PPG-2-Myristylether und PPG-3-Myristylether (Witconol^{®} APM). Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat. Weitere erfindungsgemäß bevorzugte kosmetische Öle sind ausgewählt aus den C₈₋C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI. Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol^{®} CC) oder die Ester gemäß der Lehre der DE 19756454 A1, insbesondere Glycerincarbonat. Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt. Weitere bevorzugte Öle sind verzweigte und/oder ungesättigte Carbonsäuren mit 8 - 24 C-Atomen, insbesondere mit 16 - 22 C-Atomen, in der Alkyl- oder Alkylengruppe, insbesondere Isostearinsäure, Isopalmitinsäure, Ölsäure, Linolsäure und Linolensäure, sowie Mischungen dieser Säuren.

Es kann erfindungsgemäß außerordentlich bevorzugt sein, Mischungen der vorgenannten Öle einzusetzen.

Erfindungsgemäß bevorzugte Fette sind lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, weiterhin Carbonsäuren mit 8 - 24 C-Atomen, insbesondere mit 16 - 22 C-Atomen, in der Alkylgruppe, insbesondere Laurinsäure, Myrisinsäure, Palmitinsäure, Stearinsäure, Arachinsäure und Behensäure, sowie Ester, die bei 20°C und 1013 bar fest sind, insbesondere natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat, Vaseline, hydrierte oder gehärtete Wachse, Montanesterwachse, hydrierte Jojobawachse und Sasolwachse, Polyalkylenwachse, Polyethylenglycolwachse, C₂₀-C₄₀-Dialkylester von Dimersäuren, C₃₀₋₅₀-Alkylbienenwachs, Alkyl-und Alkylarylester von Dimerfettsäuren, Ester aus einem gesättigten, einwertigen C₁₆-C₆₀-Alkohol und einer gesättigten C₈-C₃₆-Monocarbonsäure, C₁₆₋₃₆-Alkylstearate, C₁₈₋₃₈-Alkylhydroxystearoyl-stearate, C₂₀₋₄₀-Alkylerucate, Cetearylbehenat, Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 20 °C aufweisen, beispielsweise bevorzugt C₁₈ - C₃₆ Acid Triglyceride (z. B. Syncrowax^{®} HGL-C) und hydriertes Rizinusöl.

Ferner können die erfindungsgemäßen Mittel weitere Wirk-, Hilfs- und Zusatzstoffe enthalten, wie beispielsweise nichtionische Polymere, kationische Polymere, zwitterionische Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und -distearat sowie PEG-3-distearat, Pigmente, Aufhellverstärker (wie Natriumpersulfat, Kaliumpersulfat oder Ammoniumpersulfat), Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Aus Gründen der Faserschonung ist es wünschenswert, wenn die Mittel zur Farbveränderung der Haare einen möglichst neutralen pH-Wert besitzen. Zur verbesserten Farbausbildung ist jedoch ein alkalischer pH-Wert vorteilhaft. Eine weitere bevorzugte Ausführungsform der vorliegenden Erfindung besteht darin, dass das anwendungsbereite Mittel einen pH-Wert zwischen 6,0 und 12,0, bevorzugt zwischen 7,0 und 11,5, insbesondere bevorzugt zwischen 8,0 und 11,0 besitzt. Bei den pH-Werten im Sinne der vorliegenden Erfindung handelt es sich um pH-Werte, die bei einer Temperatur von 22°C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol (= Monoethanolamin), Triethanolamin, Ammoniak, 1-Amino-propan-2-ol und 2-Amino-2-methylpropan-1-ol. Die Verwendung von Ammoniak als Alkalisierungs-mittel ist besonders bevorzugt, da sich hiermit besonders gute Färbeergebnisse erzielen lassen. Das nach der Applikation auf das Haar freigesetzte Ammoniakgas kann allerdings zu Geruchsbelästigung und darüber hinaus schlimmstenfalls zur Reizung der Schleimhäute führen. Weitere bevorzugte erfindungsgemäße Zusammensetzungen enthalten daher 2-Aminoethan-1-ol als Alkali-sierungsmittel. Je nach Bedarf sind Mischungen der Alkalisierungsmittel bevorzugt, insbesondere Mischungen aus 2-Aminoethan-1-ol und 2-Amino-2-methylpropan-1-ol, Mischungen aus Ammoniak (bzw. Ammoniumhydroxid) und 2-Aminoethan-1-ol, Mischungen aus Ammoniak (bzw. Ammoniumhydroxid), 2-Aminoethan-1-ol und Natriumhydroxid, Mischungen aus Ammoniak (bzw. Ammoniumhydroxid), 2-Aminoethan-1-ol und Kaliumhydroxid sowie Mischungen aus Ammoniak (bzw. Ammoniumhydroxid), 2-Aminoethan-1-ol, Kaliumhydroxid und Natriumhydroxid.

Die Konfektionierung der erfindungsgemäßen Farbveränderungsmittel unterliegt prinzipiell keinerlei Beschränkungen. Die erfindungsgemäßen Mittel können als 1-Komponentenmittel konfektioniert werden, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der erfindungsgemäßen Komponenten separat verpackt wird.

Erfindungsgemäß bevorzugte Mittel sind dadurch gekennzeichnet, dass das Mittel unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden, und wobei ein Container ein Färbemittel enthält, welches in einem kosmetischen Träger mindestens eine farbgebende Verbindung, insbesondere ein Oxidationsfarbstoffvorprodukt, und zusätzlich mindestens ein Silicon-Blockcopolymer, enthaltend Struktureinheiten der Formeln (I) und (II) gemäß den Patentansprüchen enthält, und ein weiterer Container eine Oxidationsmittelzubereitung, enthaltend mindestens ein Oxidationsmittel, enthält.

Das anwendungsbereite Färbemittel besitzt dabei bevorzugt einen pH-Wert zwischen 5,0 und 12,0, bevorzugt zwischen 6,0 und 11,0.

Ein weiterer Gegenstand der vorliegenden Erfindung ist daher eine Mehrkomponentenverpackungseinheit (Kit-of-Parts), umfassend mindestens zwei getrennt konfektionierte Container, wobei
(i) ein Container (I) als Farbstoffzubereitung ein Mittel des ersten Erfindungsgegenstands, das heißt, eine Zusammensetzung nach einem der Ansprüche 1 bis 8 oder 11 oder 12 oder 13,
(ii) und ein weiterer Container (II) eine Oxidationsmittelzusammensetzung, enthaltend mindestens ein chemisches Oxidationsmittel, insbesondere Wasserstoffperoxid, enthält.

Für bevorzugte Ausführungsformen der erfindungsgemäßen Zusammensetzung im Container (I) gilt mutatis mutandis das vorstehend Gesagte. Für bevorzugte Ausführungsformen der Oxidationsmittelzusammensetzung im Container (II) gilt mutatis mutandis das zu bevorzugten Oxidations-mittelzusammensetzungen Gesagte.

Die Zubereitungen der Mehrkomponentenverpackungseinheit sind in getrennt voneinander konfektionierten Containern enthalten. Unter Container wird im Rahmen der vorliegenden Erfindung eine Umhüllung verstanden, die in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Es kann erfindungsgemäß vorteilhaft sein, wenn die erfindungsgemäße Mehrkomponentenverpackungseinheit mindestens ein weiteres Haarbehandlungsmittel in einem getrennten Container enthält, insbesondere ein Konditioniermittel. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Der erfindungsgemäßen Mittel werden, gegebenenfalls nach Vermischen mit einer Oxidationsmittelzubereitung in Verfahren zur Färbung keratinischer Fasern, insbesondere menschlicher Haare und bevorzugt menschlicher Kopfhaare am lebenden Anwender eingesetzt.

Solche Verfahren zur Farbveränderung menschlicher Haare sind dadurch gekennzeichnet, dass ein erfindungsgemäßes Mittel gemäß obiger Vorgaben auf das Haar aufgetragen wird, für eine Einwirkzeit von 2 bis 45 min, bevorzugt von 15 bis 30 min auf dem Haar belassen wird, und anschließend das Haar ausgespült wird.

Eine bevorzugte Ausführungsform des erfindungsgemäßen Verfahrens zur Färbung keratinischer Fasern liegt vor, wenn eine Zusammensetzung in einem kosmetischen Träger, enthaltend mindestens ein Oxidationsfarbstoffvorprodukt und zusätzlich mindestens ein Silicon-Blockcopolymer, enthaltend Struktureinheiten der Formeln (I) und (II) gemäß den Patentansprüchen, mit einer Oxidationsmittelzusammensetzung, enthaltend Wasserstoffperoxid, zu einer homogenen anwendungsbereiten Zusammensetzung vermischt, diese auf das Haar aufgebracht wird, für eine Einwirkzeit von 2 bis 120 min, bevorzugt von 3 bis 45 min auf dem Haar belassen wird, und anschließend das Haar ausgespült wird.

Eine bevorzugte Ausführungsform des erfindungsgemäßen Verfahrens zur Färbung keratinischer Fasern liegt vor, wenn eine Zusammensetzung in einem kosmetischen Träger, enthaltend mindestens ein Oxidationsfarbstoffvorprodukt und zusätzlich mindestens ein Silicon-Blockcopolymer, enthaltend Struktureinheiten der Formeln (I) und (II) gemäß den Patentansprüchen, mit einer Oxidationsmittelzusammensetzung, enthaltend Wasserstoffperoxid, bevorzugt gemäß Anspruch 9 oder 13, zu einer homogenen anwendungsbereiten Zusammensetzung vermischt, diese auf das Haar aufgebracht wird, für eine Einwirkzeit von 2 bis 120 min, bevorzugt von 3 bis 45 min auf dem Haar belassen wird, und anschließend das Haar ausgespült wird.

Eine weitere bevorzugte Ausführungsform des erfindungsgemäßen Verfahrens zur Färbung keratinischer Fasern liegt vor, wenn eine Zusammensetzung in einem kosmetischen Träger, enthaltend mindestens ein Oxidationsfarbstoffvorprodukt und zusätzlich mindestens ein Silicon-Blockcopolymer, enthaltend Struktureinheiten der Formeln (I) und (II) gemäß den Patentansprüchen, mit einer Oxidationsmittelzusammensetzung, enthaltend Wasserstoffperoxid und mindestens ein anionisches Acrylsäure- und/oder Methacrylsäure-Polymerisat oder -Copolymerisat in einer Gesamtmenge von 0,1 bis 5 Gew.-%, besonders bevorzugt von 1 bis 4 Gew.-% und insbesondere von 2 bis 3 Gew.-%, jeweils bezogen auf das Gesamtgewicht aus Farbveränderungszusammensetzung und Oxidationsmittelzusammensetzung, zu einer homogenen anwendungsbereiten Zusammensetzung vermischt, diese auf das Haar aufgebracht wird, für eine Einwirkzeit von 2 bis 120 min, bevorzugt von 3 bis 45 min auf dem Haar belassen wird, und anschließend das Haar ausgespült wird. Eine weitere bevorzugte Ausführungsform des Verfahrens zur Färbung keratinischer Fasern liegt vor, wenn eine Zusammensetzung in einem kosmetischen Träger, enthaltend mindestens ein Oxidationsfarbstoffvorprodukt, mit einer Oxidationsmittelzusammensetzung, enthaltend Wasserstoffperoxid und zusätzlich mindestens ein Silicon-Blockcopolymer, enthaltend Struktureinheiten der Formeln (I) und (II) gemäß den Patentansprüchen, zu einer homogenen anwendungsbereiten Zusammensetzung vermischt, diese auf das Haar aufgebracht wird, für eine Einwirkzeit von 2 bis 120 min, bevorzugt von 3 bis 45 min auf dem Haar belassen wird, und anschließend das Haar ausgespült wird.

Eine weitere bevorzugte Ausführungsform des Verfahrens zur Färbung keratinischer Fasern liegt vor, wenn eine Zusammensetzung in einem kosmetischen Träger, enthaltend mindestens ein Oxidationsfarbstoffvorprodukt, mit einer Oxidationsmittelzusammensetzung, enthaltend Wasserstoffperoxid, mindestens ein Silicon-Blockcopolymer, enthaltend Struktureinheiten der Formeln (I) und (II) gemäß den Patentansprüchen und zusätzlich mindestens ein anionisches Acrylsäure-und/oder Methacrylsäure-Polymerisat oder -Copolymerisat in einer Gesamtmenge von 0,1 bis 5 Gew.-%, besonders bevorzugt von 1 bis 4 Gew.-% und insbesondere von 2 bis 3 Gew.-%, jeweils bezogen auf das Gesamtgewicht aus Farbveränderungszusammensetzung und Oxidationsmittelzusammensetzung, zu einer homogenen anwendungsbereiten Zusammensetzung vermischt, diese auf das Haar aufgebracht wird, für eine Einwirkzeit von 2 bis 120 min, bevorzugt von 3 bis 45 min auf dem Haar belassen wird, und anschließend das Haar ausgespült wird.

Die Anwendungstemperaturen bei der erfindungsgemäßen Farbveränderung keratinischer Fasern können in einem Bereich zwischen 15 und 45°C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Bezüglich weiterer bevorzugter Ausführungsformen der erfindungsgemäßen Verfahren gilt mutatis mutandis das zu den erfindungsgemäßen Mitteln Gesagte.

Durch Anwendung der erfindungsgemäßen Mittel zur oxidativen Färbung menschlicher Haare wird eine verbesserte Haaroberfläche gegenüber vergleichbaren oxidativen Färbemitteln erzielt. Ebenso werden eine vergleichsweise verringerte Haarstrukturschädigung, eine verbesserte Haaroberfläche und damit eine verbesserte Pflege der Haarfasern beobachtet. Diese Wirkungen lassen sich durch einen verbesserten Glanz und durch eine verbesserte Kämmbarkeit der gefärbten Haare darstellen. Ein weiterer Erfindungsgegenstand ist daher die Verwendung von Silicon-Blockcopolymeren b) enthaltend Struktureinheiten der Formeln (I) und (II) gemäß den Patentansprüchen wie vorstehend beschrieben,
- zur Verbesserung des Glanzes gefärbter keratinischer Fasern und/oder
- zum Schutz der keratinischen Fasern vor oxidativen Schädigungen und/oder
- zur Erhöhung der Waschbeständigkeit gefärbter keratinischer Fasern und/oder
- zur Verbesserung der Kämmbarkeiten gefärbter keratinischer Fasern und/oder
- zur Verbesserung des Griffs gefärbter keratinischer Fasern und/oder
- zur Verbesserung der statischen Eigenschaften gefärbter keratinischer Fasern
- zur Verringerung der Schädigung der Haarstruktur bei der oxidativen Farbveränderung menschlicher Haare.

Ein weiterer Erfindungsgegenstand ist die Verwendung der Mittel des ersten Erfindungsgegenstands zur Verbesserung der Haaroberfläche und zur Verringerung der Schädigung der Haarstruktur bei der oxidativen Farbveränderung menschlicher Haare.

Bezüglich weiterer bevorzugter Ausführungsformen der erfindungsgemäßen Verwendungen gilt mutatis mutandis das zu den erfindungsgemäßen Mitteln Gesagte.

### Beispiele (alle Mengenangaben in Gew.-%)

### 1.1) Zubereitungen: Färbecremes (Tabelle 1)

| Rohstoff | E | V1 | V2 | V3 | V4 |
|---|---|---|---|---|---|
| Lanette D [1] | 17,9 | 17,9 | 17,9 | 17,9 | 17,9 |
| 2-Octyldodecanol | 2,3 | 2,3 | 2,3 | 2,3 | 2,3 |
| Xanthan | 0,05 | 0,05 | 0,05 | 0,05 | 0,05 |
| Glycerinmonostearat | 6,0 | 6,0 | 6,0 | 6,0 | 6,0 |
| Kokosamidopropylbetain, 40%ig | 2,0 | 2,0 | 2,0 | 2,0 | 2,0 |
| Monoethanolamin | 4,5 | 4,5 | 4,5 | 4,5 | 4,5 |
| 2-Amino-2-methylpropanol | 0,1 | 0,1 | 0,1 | 0,1 | 0,1 |
| p-Toluylendiaminsulfat | 1,25 | 1,25 | 1,25 | 1,25 | 1,25 |
| Resorcin | 0,35 | 0,35 | 0,35 | 0,35 | 0,35 |
| 3-Aminophenol | 0,1 | 0,1 | 0,1 | 0,1 | 0,1 |
| 4-Chlorresorcin | 0,25 | 0,25 | 0,25 | 0,25 | 0,25 |
| Silikon-Blockcopolymer [2] | 1,0 | - | - | - | - |
| Polyquaternium-2 | - | - | 1,0 | - | - |
| Polyquaternium-39 | - | - | - | 1,0 | - |
| Dimethicon | - | - | - | - | 1,0 |
| Parfum | qs | qs | qs | qs | qs |
| Wasser, entsalzt | ad 100 | | | | |

| | | | | | |
|---|---|---|---|---|---|
| [1] C₁₆-C₁₈-Fettalkohol (INCI-Bezeichnung: Cetearyl alcohol) (BASF) [2] enthaltend Struktureinheiten der Formeln (Ib) und (IIa) mit k=14 | | | | | |

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

Die erfindungsgemäße Zusammensetzung E enthält ein Silicon-Blockcopolymer mit Struktureinheiten der Formeln (Ib) und (IIa) mit k=14, während die Vergleichszusammensetzung V1 keinen Pflegestoff enthält. Die Vergleichzusammensetzungen V2 bis V4 enthalten übliche Pflegestoffe (PQ-2 bzw. PQ-39 bzw. Silikon).

### 1.2) Entwicklerzubereitung EW (Tabelle 2)

| Rohstoff | Gew.-% |
|---|---|
| Dipicolinsäure | 0,10 |
| Dinatriumpyrophosphat | 0,03 |
| HEDP, 60% | 1,50 |
| Texapon NSO [3] | 2,00 |
| Dow Corning DB 110 A [4] | 0,07 |
| Aculyn 33A [5] | 15,00 |
| Ammoniak, 25 % | 0,65 |
| Wasserstoffperoxid, 50 % | 12,00 |
| Wasser, vollentsalzt | ad 100 |

| | |
|---|---|
| [3] Laurylalkohol-diglykolethersulfat, Na-Salz (ca. 28%, INCI-Bezeichnung: Sodium Laureth Sulfate) (BASF) [4] nicht-ionische Silikonemulsion (INCI-Bezeichnung: Dimethicon) (Dow Corning) [5] Acrylpolymer (ca. 28% in Wasser; INCI-Bezeichnung: Acrylates Copolymer) (Rohm & Haas) | |

Die Färbecremes wurden vor der Anwendung mit der Entwicklerlösung EW im Gewichtsverhältnis von 1:1 versetzt und innig vermischt. Pro Gramm Haarsträhne (Kerling International, Backnang (DE); europäisches, natürliches Humanhaar 9/0 bzw. 7/0) wurden 4 g des frisch hergestellten, anwendungsbereiten Färbemittels aufgetragen. Die Einwirkzeit betrug 30 min bei 35°C für die Färbemittel. Danach wurden die Strähnen 30 s lang mit warmem Wasser ausgespült und luftgetrocknet.

### 2) Ergebnisse

### 2.1) Nasskämmbarkeiten

Zur Beurteilung der Nasskämmarbeit wurden jeweils 12 Haarsträhnen (Kerling International (Backnang), Euro-Naturhaar 7/0) mit einem anwendungsbereiten Mittel ausgefärbt.

Nach Anwendung der Färbemittel wurden die Strähnen mit Wasser für 2 min gespült und die wassernassen Strähnen 10x automatisch mit Hartgummikämmen (Hercules Sägemann) gekämmt und die aufzuwendende Arbeit bestimmt. Es wurden jeweils 12 Haarsträhnen behandelt und gemessen. Als Wert für die Kämmarbeit wurde jeweils das arithmetische Mittel gebildet.

Folgende Ergebnisse wurden erhalten (Tabelle 3):

| eingesetztes Färbemittel | Nasskämmarbeit [mJ] |
|---|---|
| E1 + EW (erfindungsgemäß) | 269 |
| V1 + EW (Vergleich) | 668 |
| V2 + EW (Vergleich) | 651 |
| V3 + EW (Vergleich) | 672 |
| V4 + EW (Vergleich) | 642 |

Die Ergebnisse zeigen eine deutliche Verbesserung der Nasskämmbarkeit durch die erfindungsgemäßen Färbemittel.

### 2.2) Trockenkämmbarkeiten

Zur Beurteilung der Trockenkämmarbeit wurden jeweils 12 Haarsträhnen (Kerling International (Backnang), Euro-Naturhaar 7/0) mit einem anwendungsbereiten Mittel ausgefärbt.

Nach Anwendung der Färbemittel wurden die Strähnen mit Wasser für 2 min gespült und die luftgetrockneten Strähnen 10x automatisch mit Hartgummikämmen (Hercules Sägemann) gekämmt und die aufzuwendende Arbeit bestimmt. Es wurden jeweils 12 Haarsträhnen behandelt und gemessen. Als Wert für die Kämmarbeit wurde jeweils das arithmetische Mittel gebildet.

Folgende Ergebnisse wurden erhalten (Tabelle 4):

| eingesetztes Färbemittel | Trockenkämmarbeit [mJ] |
|---|---|
| E1 + EW (erfindungsgemäß) | 153 |
| V1 + EW (Vergleich) | 241 |
| V2 + EW (Vergleich) | 272 |
| V3 + EW (Vergleich) | 232 |
| V4 + EW (Vergleich) | 274 |

Die Ergebnisse zeigen eine deutliche Verbesserung der Trockenkämmbarkeit durch die erfindungsgemäßen Färbemittel.

## Patentansprüche

1. Zusammensetzung zur Farbveränderung keratinischer Fasern, enthaltend
a) mindestens eine Verbindung aus der Gruppe der Oxidationsfarbstoffvorprodukte, der direktziehenden Farbstoffe, Wasserstoffperoxid oder deren Mischungen
b) mindestens ein Silicon-Blockcopolymer, enthaltend Struktureinheiten der Formeln (I) und (II) 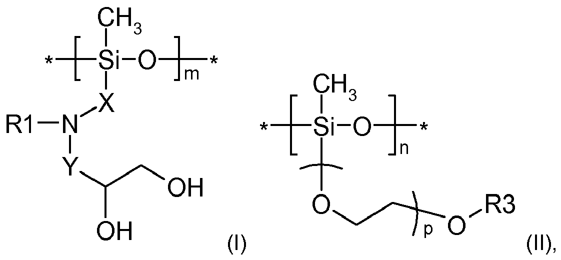 in denen
X für einen substituierten oder unsubstituierten Alkylenrest mit 1, 2, 3, 4, 5, 6, 7 oder 8 C-Atomen steht,
Y für einen substituierten oder unsubstituierten Alkylenrest mit 1, 2, 3, 4, 5, 6, 7 oder 8 C-Atomen steht,
R1 für einen Methyl-, Ethyl-, n-Propyl-, Isopropyl-, n-Butyl-, sec-Butyl, Isobutyl- oder tert-Butylrest steht,
R3 für -H, einen Methyl-, Ethyl-, n-Propyl-, Isopropyl-, n-Butyl-, sec-Butyl, Isobutyl- oder tert-Butylrest steht,
p für eine ganze Zahl von 1 bis 30, vorzugsweise für 10, 11, 12, 13, 14, 15, 16, 17 oder 18 steht
m und n für ganze Zahlen zwischen 2 und 200 stehen.

2. Zusammensetzung nach Anspruch 1, **dadurch gekennzeichnet, dass** sie mindestens ein Silicon-Blockcopolymer enthält, das Struktureinheiten der Formel (la) 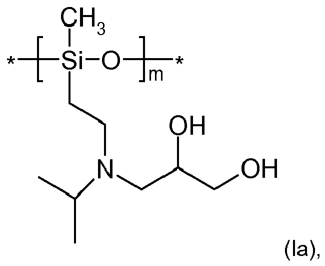 enthält, in der m für eine ganze Zahl zwischen 2 und 200 steht.

3. Zusammensetzung nach einem der Ansprüche 1 oder 2, **dadurch gekennzeichnet, dass** sie mindestens ein Silicon-Blockcopolymer enthält, das Struktureinheiten der Formel (IIa) 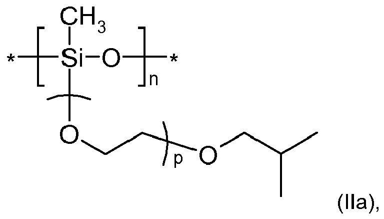 enthält, in der n für eine ganze Zahl zwischen 2 und 200 und p für die Zahl 10, 11, 12, 13, 14, 15 oder 16, vorzugsweise für 14, steht.

4. Zusammensetzung nach einem der Ansprüche 1 bis 3, **dadurch gekennzeichnet, dass** sie - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% Silicon-Blockcopolymer(e), enthaltend Struktureinheiten der Formeln (I) und (II), enthält.

5. Zusammensetzung nach einem der Ansprüche 1 bis 4, **dadurch gekennzeichnet, dass** sie - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% 2-Butyloctan-1-ol (INCI-Bezeichnung: Butyloctanol) enthält.

6. Zusammensetzung zur Farbveränderung keratinischer Fasern, enthaltend a) mindestens eine Verbindung aus der Gruppe der Oxidationsfarbstoffvorprodukte, der direktziehenden Farbstoffe, Wasserstoffperoxid oder deren Mischungen b) mindestens eine Verbindung mit der INCI-Bezeichnung Bis-Isopropylamino-PG-Propyl Dimethicone/Bis-Isobutyl PEG-14 Copolymer.

7. Zusammensetzung nach einem der Ansprüche 1 bis 6, **dadurch gekennzeichnet, dass** es als Oxidationsfarbstoffvorprodukt mindestens eine Entwicklerkomponente und/oder mindestens eine Kupplerkomponente enthält, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'- diaminophenyl)-1,4,7,10-tetraoxadecan, 4-Aminophenol, 4- Amino-3-methylphenol, Bis-(5- amino-2-hydroxy-phenyl)methan, 2,4,5,6- Tetraaminopyrimidin, 2-Hydroxy-4,5,6- triaminopyrimidin, 4,5-Diami-no-1-(2-hydroxyethyl)-pyrazol und bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

8. Zusammensetzung nach einem der Ansprüche 1 bis 7, **dadurch gekennzeichnet, dass** es mindestens einen direktziehenden Farbstoff enthält, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitro-benzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)-amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

9. Zusammensetzung nach einem der Ansprüche 1 bis 8, **dadurch gekennzeichnet, dass** es als Blondiermittel oder Entwickler konfektioniert ist und - bezogen auf sein Gewicht - 0,5 bis 15 Gew.-%, vorzugsweise 0,75 bis 10 Gew.-%, besonders bevorzugt 1 bis 7,5 Gew.-%, weiter bevorzugt 1,25 bis 7 Gew.-% und insbesondere 1,5 bis 6,0 Gew.-% Wasserstoffperoxid (berechnet als 100 %iges H₂O₂) enthält.

10. Verwendung von Silicon-Blockcopolymeren b), enthaltend Struktureinheiten der Formeln (I) und (II) 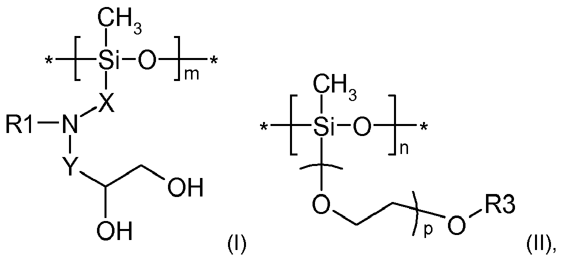 in denen
X für einen substituierten oder unsubstituierten Alkylenrest mit 1, 2, 3, 4, 5, 6, 7 oder 8 C-Atomen steht,
Y für einen substituierten oder unsubstituierten Alkylenrest mit 1, 2, 3, 4, 5, 6, 7 oder 8 C-Atomen steht,
R1 für einen Methyl-, Ethyl-, n-Propyl-, Isopropyl-, n-Butyl-, sec-Butyl, Isobutyl- oder tert-Butylrest steht,
R3 für -H, einen Methyl-, Ethyl-, n-Propyl-, Isopropyl-, n-Butyl-, sec-Butyl, Isobutyl- oder tert-Butylrest steht,
p für eine ganze Zahl von 1 bis 30, vorzugsweise für 10, 11, 12, 13, 14, 15, 16, 17 oder 18 steht
m und n für ganze Zahlen zwischen 2 und 200 stehen,
gemäß einem der Ansprüche 1, 2, 3, 4, 5 oder 6
- zur Verbesserung des Glanzes gefärbter keratinischer Fasern und/oder
- zum Schutz der keratinischen Fasern vor oxidativen Schädigungen und/oder
- zur Erhöhung der Waschbeständigkeit gefärbter keratinischer Fasern und/oder
- zur Verbesserung der Kämmbarkeiten gefärbter keratinischer Fasern und/oder
- zur Verbesserung des Griffs gefärbter keratinischer Fasern und/oder
- zur Verbesserung der statischen Eigenschaften gefärbter keratinischer Fasern
- zur Verringerung der Schädigung der Haarstruktur bei der oxidativen Farbveränderung menschlicher Haare.

11. Zusammensetzung nach einem der Ansprüche 1 bis 9, **dadurch gekennzeichnet, dass** zusätzlich mindestens ein Fett und/oder mindestens ein Öl in einer Gesamtmenge von 0,1 bis 90 Gew.-%, bevorzugt 1,0 bis 80 Gew.-%, besonders bevorzugt 5,0 bis 60 Gew.-%, außerordentlich bevorzugt 10 bis 45 Gew.-%, jeweils bezogen auf das Gewicht der Zusammensetzung, enthalten ist.

12. Zusammensetzung nach einem der Ansprüche 1 bis 9 oder 11, **dadurch gekennzeichnet, dass** ein Alkalisierungsmittel enthalten ist, das ausgewählt ist aus 2-Aminoethan-1-ol, Mischungen aus 2-Aminoethan-1-ol und 2-Amino-2-methylpropan-1-ol, Mischungen aus Ammoniak (bzw. Ammoniumhydroxid) und 2-Aminoethan-1-ol, Mischungen aus Ammoniak (bzw. Ammoniumhydroxid), 2-Aminoethan-1-ol und Natriumhydroxid, Mischungen aus Ammoniak (bzw. Ammoniumhydroxid), 2-Aminoethan-1-ol und Kaliumhydroxid sowie Mischungen aus Ammoniak (bzw. Ammoniumhydroxid), 2-Aminoethan-1-ol, Kaliumhydroxid und Natriumhydroxid.

13. Zusammensetzung nach Anspruch 9, **dadurch gekennzeichnet, dass** es als Blondiermittel oder Entwickler konfektioniert ist und zusätzlich mindestens ein anionisches Acrylsäure- und/oder Methacrylsäure-Polymerisat oder -Copolymerisat in einer Gesamtmenge von 0,1 bis 5 Gew.-%, besonders bevorzugt von 1 bis 4 Gew.-% und insbesondere von 2 bis 3 Gew.-%, jeweils bezogen auf das Gewicht der Zusammensetzung, enthalten ist.

14. Mehrkomponentenverpackungseinheit (Kit-of-Parts), umfassend mindestens zwei getrennt konfektionierte Container, wobei
i) ein Container (I) als Farbstoffzubereitung eine Zusammensetzung nach einem der Ansprüche 1 bis 8 oder 11 oder 12 oder 13
und
ii) ein weiterer Container (II) eine Oxidationsmittelzusammensetzung, enthaltend mindestens ein chemisches Oxidationsmittel, insbesondere Wasserstoffperoxid, enthält.

15. Verfahren zur Färbung keratinischer Fasern bei dem eine Zusammensetzung in einem kosmetischen Träger, enthaltend mindestens ein Oxidationsfarbstoffvorprodukt und zusätzlich mindestens ein Silicon-Blockcopolymer, enthaltend Struktureinheiten der Formeln (I) und (II)gemäß den Patentansprüchen 1, 2, 3, 4, 5 oder 6, mit einer Oxidationsmittelzusammensetzung, enthaltend Wasserstoffperoxid, bevorzugt gemäß Anspruch 9 oder 13, zu einer homogenen anwendungsbereiten Zusammensetzung vermischt, diese auf das Haar aufgebracht wird, für eine Einwirkzeit von 2 bis 120 min, bevorzugt von 3 bis 45 min auf dem Haar belassen wird, und anschließend das Haar ausgespült wird.
